(19) Europäisches Patentamt
European Patent Office
Office européen des brevets

(11) Publication number: 0 139 373 A1

# (12) EUROPEAN PATENT APPLICATION

(21) Application number: 84305455.2

(22) Date of filing: 10.08.84

(51) Int. Cl.[4]: G 01 N 33/543
G 01 N 33/535, G 01 N 33/532
G 01 N 33/483

(30) Priority: 26.08.83 US 526970

(43) Date of publication of application:
02.05.85 Bulletin 85/18

(84) Designated Contracting States:
AT BE CH DE FR GB IT LI LU NL SE

(71) Applicant: THE REGENTS OF THE UNIVERSITY OF CALIFORNIA
2199 Addison Street
Berkeley California 94720(US)

(72) Inventor: Christian, Clifford N.
3081 Moloki Place
Costa Mesa California 92626(US)

(74) Representative: Dealtry, Brian et al,
Eric Potter & Clarkson 14, Oxford Street
Nottingham NG1 5BP(GB)

## (54) Multiple immunoassay system.

(57) A microassay rod adapted for use in screening of biologic fluids and other sample fluids or gases for the presence of substances which are capable of undergoing specific binding reactions. The microassay rod is based on a column of spaced detection layers with each detection layer including a binding amount of a known binding agent. The binding agent is capable of specifically binding with the specific substance being tested for. The detection layers are spaced apart and positioned by interleaved spacer layers. Up to 250 and more detection layers may be formed into a column to simultaneously screen a test solution for substances which are reactive or binding with the reagent present in each individual detection layer. The assay rod has wide application for use in immunoabsorbent assay systems and is especially well suited for Enzyme-Linked Immunosorbent Assay systems. A magnetic marker is also disclosed for use in detecting an antibody-antigen reaction. Use of the microassay rod in multiple microassay apparatus is also disclosed.

EP 0 139 373 A1

Croydon Printing Company Ltd.

# MULTIPLE IMMUNOASSAY SYSTEM

## Background of the Invention

The present invention relates generally to procedures and systems for immunoabsorbent assay. More particularly, the present invention relates to a micro immunoassay system which is capable of testing a single small sample simultaneously for the presence of numerous different biologically important substances.

Immunoassay systems are all based upon the specific yet reversible chemical reaction which occurs between an antigen and a corresponding antibody. In general, a known antigen or antibody is absorbed onto or otherwise attached to a solid support. The fixed antigen or antibody is then used to screen or probe various solutions of interest to detect the presence of antibodies or antigens which are immunoreactive with the bound antigen or antibody.

For example, if a test solution is to be screened or probed for a particular antibody, a known antigen which combines specifically with the antibody to be tested for is attached to a suitable support structure. The fixed known antigen is then used to probe a test solution for the presence of the antibody. If the specific antibody is present, it will combine with the fixed antigen to thereby become indirectly bound to the fixed support material. Any unbound antibody and other interferring substances are then removed from the support surface by washing. The presence of any antibody indirectly bound to the fixed support by way of the known antigen is then detected by the use of a second antibody which has a particular characteristic, or includes a suitable marker which can be measured.

Many different types of antibody markers have been developed for use in immunoassay systems. Antibodies tagged with radioisotopes have been popular markers for use in radioimmunoassay; however, the use of radioisotopes requires expensive specialized equipment and specialized handling features. Enzyme Linked Immunoabsorbent Assay (ELISA) is another immunoassay system which has experienced wide spread popularity. ELISA is based upon the use of an enzyme linked antibody marker to detect the presence of antibody bound to the fixed antigen. Typically, an enzyme such as horseradish peroxidase is linked to an immunoglobulin which is capable of combining with the antibody being tested for. The enzyme is capable of catalyzing an easily measured color producing reaction. The popularity of ELISA type procedures is based in large part upon the ease with which the colored product produced by the enzyme-linked antibody can be detected. In addition to radioisotope and enzyme linked antibody markers, fluorescent compounds, such as fluorescein have been used to provide a fluorescent antibody marker.

With the advent of monoclonal antibodies, and highly specific antigens, it is now possible using immunoassay techniques to probe various test solutions such as blood, sputum or urine specifically and selectively for numerous different immunochemically reactive substances, i.e. those substances capable of engaging in antibody-antigen type reactions. It would be desirable to use these newly available immunochemical probes to provide an assay system in which a large number of specific antibodies or antigens could be screened for simultaneously using a single aliquot of sample and a single test system.

For the most part, the present immunoassay systems utilize a single solid support coated with a single known antigen or antibody. The solid support is then used to probe one or more samples for a single immunoreactive substance (i.e. antigen or antibody). When screening a test solution for more than one antigen or antibody, the test solution must be separated to provide separate aliquots for testing. This type of screening procedure is especially undesirable where a large number of antigens or antibodies are being tested for and where the amount of test solution available is small.

In 1982, Hawkes et al. published a paper describing a Dot-Immunobinding Assay (Hawkes, R., Niday, E., and Gordon, J., Analytical Biochemistry 119, 142-147 (1982)) which provides for the simultaneous probing or screening of a test solution for a number of antibodies. The Dot-Immunobinding Assay basically involves dotting a number of different antigens in different places on the surface of a nitrocellulose filter paper. Upon drying, the antigens are bound non-covalently to the paper and remain attached to it during subsequent manipulations. This single piece of filter paper is then used as the solid support to simultaneously test for the presence of antibodies reactive with the dotted antigens. An enzyme-linked antibody and associated chromogenic substrate for the enzyme is then used to "develop" the filter paper to detect the presence of antibodies bound to the dotted antigens on the filter paper.

The Dot-Immunobinding Assay system is well suited for screening a test solution for a relatively small number of antigen or antibodies. Difficulties arise, however, when a large number of antigens or antibodies are to be tested for. Such difficulties include problems in spotting a relatively large number of well defined (i.e. non-overlapping) dots of known antigens or antibody on a single filter paper. Problems with reading the closely spaced dots are also expected. If the dots are spaced apart on the filter paper to maintain dot integrity, it is then necessary to use an inordinately large sample of test solution to conduct a simultaneous analysis since a relatively large area of filter paper must be treated with the solution to be tested.

Accordingly, there is still a continuing need to provide a multiple immunoassay system which can conveniently, quickly, efficiently and accurately screen a microsized sample for a large number of antigens and/or antibodies.

Although the radioisotope markers, enzyme linked markers and fluorescent markers are all well suited for immunoabsorbent assay of individual antibodies, it would be desirable to provide a new marker or tag which can be used in a multiple immunoassay system to provide accurate and quick identification of a large number of discrete bound antibodies. It would also be desirable to provide a marker whose characteristic can be easily measured and interfaced with computer processing equipment.

## Summary of the Invention

In accordance with the present invention procedures and systems are disclosed which provides for the simultaneous testing of a single small sample for the presence of numerous different biologically important substances. The multiple micro assay system in accordance with the present invention can be used to detect and quantify substances which engage in a binding reaction, including the following: Antigen-antibody, nucleic acid hydridization, and receptor-ligand. In general, the system can be used to detect any substance if the material to which it specifically binds can be attached to a solid support. As features of the present invention, a single sample solution can be tested simultaneously for the presence of up to 250 and even more substance, such as antigens or antibodies. The steps involved in the present invention are no more time consuming or cumbersome than for conventional assay of a single substance. The assay system can be read and quantified automatically in less than one second. The system is especially well suited for use with any of the conventional antibody markers, used in immunoabsorbent assays, with the exception of radioactive probes.

One aspect of the present invention is based upon an immunoassay rod which is basically a column of spaced detection layers which are laminated or otherwise bound between alternating spacer layers. Each detection layer includes a known immunoreactive reagent such as a known antigen or known antibody. The immunoassay rod in accordance with the present invention is based upon the same principles as conventional immunoassay systems except that the spaced stacking of the detection layers provides an especially convenient and simple means for simultaneously probing a single solution for a multitude of immunochemically reactive substances. The number of antibodies or antigens which can be simultaneously tested for is quite large. For example, up to 250 and even more antigens or antibodies can be screened for simultaneously by simply preparing an immunoassay rod in accordance with the present invention having the desired number of detection layers.

As a feature of the present invention, the results of an assay carried out using the multiple assay rod can be automatically read by a computer or other suitable device with the evaluation taking no more than one second for all 250 assays. This is a substantial improvement over prior art techniques where test results have to be individually read and recorded. As another feature of the present invention, no more sample or reagents are used during the test for the presence of a large number of different substances than is presently used in prior art procedures for testing the presence of one substance. This feature is especially desirable where expensive reagents are used or where only a small amount of sample is available.

As another aspect of the present invention, a new type of antibody-marker is disclosed which is especially well suited for use with the multiple immunoassay rod system. The new marker in its broadest aspect is a magnetizable marker which can be linked to a suitable antibody for use in detecting the presence of bound antibody or antigens. The use of a magnetizable marker is especially well suited for multiple immunoassay since the magnetizable marker can be detected electrically and input directly into a computer system for data processing and printout.

The above discussed features and attendant advantages of the present invention will become better understood by reference to the following description when considered in conjunction with the accompanying drawings.

Brief Description of the Drawings

Fig. 1 is an enlarged view of a preferred immunoassay rod in accordance with the present invention.

Fig. 2 is a side view of the preferred immunoassay rod inside a support tube.

Fig. 3 is a cross-sectional view of Fig. 2 taken in the III-III plane.

Fig. 4 is a representation of a step in the preferred fabrication of an immunoassay rod in accordance with the present invention.

Fig. 5 is a representation of an additional step in fabrication of a preferred immunoassay rod in accordance with the present invention.

Fig. 6 is a representation of the final preferred cutting step used in preparation of an immunoassay rod in accordance with the present invention.

Fig. 7 is a schematic representation of a preferred exemplary system for reading the immunoassay rod.

Fig. 8 is a prospective view of an alternate preferred immunoassay rod shown in position within another preferred support structure.

Fig. 9 is a schematic representation of a reading system designed to detect magnetizable markers when such markers in accordance with the present invention are used to detect the presence of antibodies bound to the various detection layers.

Fig. 10 is a partial perspective view of a preferred manifold and microtiter plate used for testing multiple samples simultaneously.

Detailed Description of the Invention

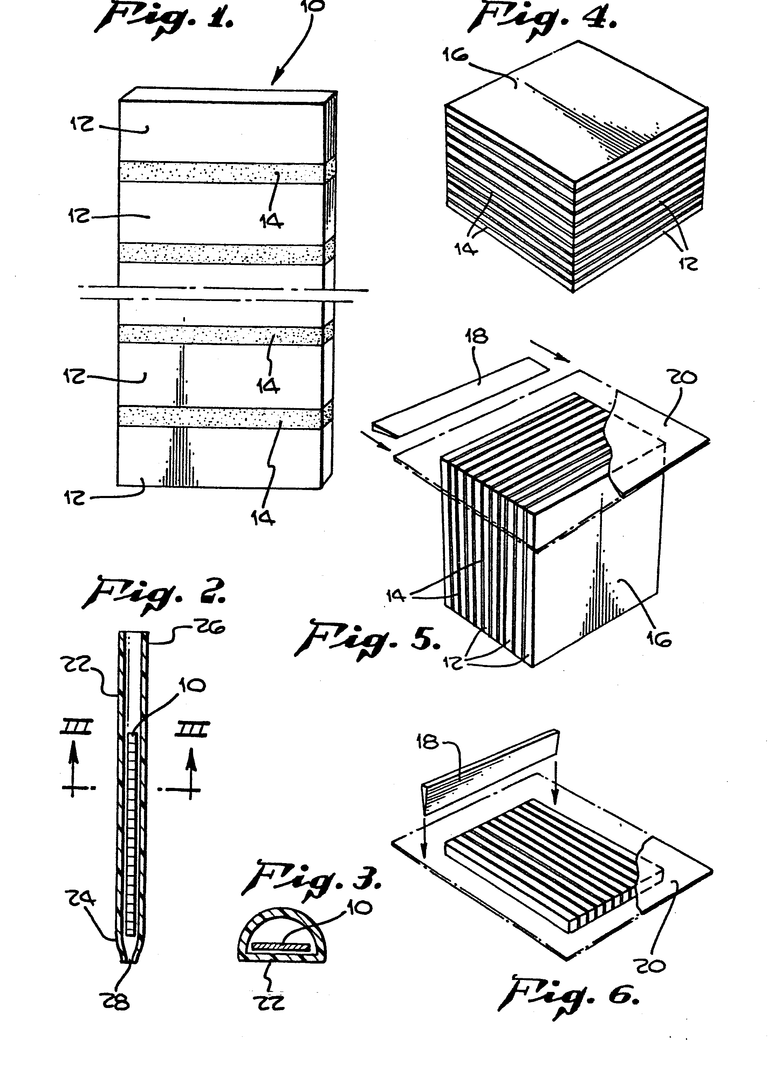

A preferred exemplary embodiment of a microassay rod in accordance with the present invention is shown generally at 10 in Fig. 1. The rod 10 is basically a column having a plurality of detection layers 12 which are positioned and spaced apart by alternating spacer layers 14. Each detection layer 12 may include one or more reagents which can react with or otherwise provide for the detection of a desired substance. The spacer layers 14 maintain detection layers 12 at spaced location so the immunoassay carried out in each individual detection layer may be discretely measured by a suitable detection system.

The rod 10 in accordance with the present invention has wide application to any type of assay or spot test where a detection reagent is placed on a suitable absorbent for use in detecting the presence of a desired substance. For example, the assay rod can be used for carrying out conventional color spot tests used in qualitative detection of inorganic and/or organic substances. As mentioned above, the multiple microassay system can be used to detect and quantify substances which engage in a binding reaction, including the following: antigen-antibody, nucleic acid hybridization, and receptor-ligand. For example, one use for rod 10 applies the conventional techniques of nucleic acid hydridization to nucleic acids attached to nitrocellulose filters. Single strands of either DNA or RNA can be attached to the solid support and used as a detector layer for complimentary nucleic acid copies. Colorimetric or magnetic methods are then used to detect the hybridization. An additional use incorporates biological receptors onto a solid support for the purpose of detecting the presence of receptor binding substances such as hormones, transmitters, and synthetic ligands.

Although the assay rod in accordance with the present invention has wide application to the detection of substances in general, it is especially well suited for use in immunoassay systems. The following detailed description will be limited to the discussion of preferred exemplary embodiments of the assay rod in connection with immunoassay with it being understood that the present invention has wide application to detection systems not based on immunochemical (antibody-antigen) reactions.

In a preferred embodiment, the assay rod 10 is used for conventional ELISA detection of antibodies or antigens. Each detection layer 12 is preferably made from a suitable absorbent support material which is capable of binding known antigens or antibodies. Typical detection layer materials are nitrocellulose filter paper, plastics, various fibers such as cotton, wool, and numerous synthetic fibers, including nylon, and any other suitable support material which does not prevent immunochemical reactions. Standard nitrocellulose filter paper (e.g. Millipore AA type, pore size from 0.2 to 1.2 microns, or equivalent) is preferred.

The spacer layers 14 can be made from any suitable material which provides separation and positioning of the detection layers 12. It is not necessary that the spacer layers 14 be impervious to water or that it prevent the passage of antigens or antibodies between the various detection layers. It is only required that the spacer layers 14 be inert with respect to antigens and antibodies and that it provide suitable structural positioning and spacing of the detection layers 12. The spacer layers 14 may be made from any of the same materials used for the detection layers 12.

Means must be provided for adhering the spacer layers 14 to the detection layers 12 to provide a laminated rod structure. Any suitable adhesive may be used to stick the detection and spacer layers together. In addition to using adhesives to produce a laminated column of alternating detection and spacer layers, suitable thermoplastic materials may be used as the spacer layers. These thermoplastic materials when heated stick to the detection layers to provide a laminated column of alternating detection and spacer layers.

The preferred assay rod 10 will have a rectangular cross section having a width of between 0.001 inch and 0.005 inch and a length of from 0.010 inch to 0.03 inch. The length of the assay rod will vary depending upon the number of detection layers 12 desired. Rods with up to 250 detection layers are preferred with rods having more than 250 detection layers being possible. Preferably the detection layers will be from 0.002 to 0.006 inch thick with the spacer layers being from 0.001 to 0.005 inch thick.

As previously mentioned, the assay rod in accordance with the present invention may be used for conventional ELISA detection of either antigens or antibodies. The following description of the fabrication of a preferred assay rod will be limited to a rod in which antigens are fixed to the detection layers 12 for use in detecting the presence of antibodies in test solutions.

Standard Millipore AA type nitrocellose filter paper having pores sized from 0.2 to 1.2 microns is cut into one inch squares. For each antibody to be assayed or probed for, its specific antigen is non-covalently absorbed to a single nitrocellulose filter paper square.

Preferably, the antigen is in solution and is absorbed onto the filter paper and then dried. Antigens which cannot be bound directly to nitrocellulose filter paper can be covalently linked to chemically derivatized paper which e.g. is commercially available from Schleicher & Schuell and marketed under the name Transbind.

The filter paper squares (with absorbed antigens) are then stacked with interleaved squares of colored plastic available from Dow Chemical Corp. under the trade name Surlyn 1601. The colored squares of plastic are preferably 0.002 inch thick. The stack of alternating filter paper and plastic layers is compressed and heated to 90 degrees centigrade for 30 minutes. The plastic becomes sticky at this temperature, creating a laminated block of filter papers separated by the opaque colored plastic. If antigens which are inactivated by temperature are being used, a lower melting point plastic such as Suryln 1856 can be used. Alternatively, double sided adhesive tape may be substituted for the thermoplastic spacers. The stacking of the alternating filter paper and plastic layers is shown in Fig. 4.

As shown in Fig. 5, the laminated block 16 of alternating filter paper and plastic layers is positioned in a microtome with the layers being at a right angle to microtome blade 18. The block 16 is sectioned to produce 0.002 inch sheets of alternating filter paper and plastic layers. Before each cut, an adhesive tape 20 such as Scotch tape, 3M or equivalent is applied to the block face as shown in Fig. 5 to stabilize each section and serve as a backing for the assay rod through subsequent manipulations. Alternatively, a film of clear plastic can be heat sealed to the block face instead of adhesive tape. This step yields approximately 500 pieces of tape to which are attached 0.002 inch thick sheets having lines of alternating antigen impregnated filter paper and plastic layers.

As shown in Fig. 6, the sectioned sheets with alternating impregnated filter paper and plastic layers are further sectioned into 0.020 inch intervals. The sections are cut at right angles to the lines of filter paper and plastic to thereby produce 50 rods from each section. Thus, the total number of rods which can be produced from the one inch square stack of filter papers is 50 X 500 = 25,000. As is apparent, this method of fabricating rods is an especially convenient and quick means for preparing a large number of rods which each are capable of screening for a large number of antibodies.

The assay rod 10 is preferably placed within a suitable protective structure for use. As shown in Figs. 2 and 3, the rod 10 is preferably placed within a support tube 22. The support tube 22 may be made from glass, plastic or any suitable structurally strong material which is of optical quality. The tube 22 must be optically clear to prevent any interference with optical reading of the assay rod. The tube 22 has a bottom portion 24 and a top portion 26. The bottom portion 24 includes an opening 28 through which test solution may be drawn for application to the assay rod 10. Preferably, a vacuum apparatus or other suction device is attached to the tube upper support portion 26 to provide the necessary vacuum to draw solution up through opening 28. Other suitable support tubes and channel structures are possible so long as they provide adequate support and protection for the assay rod while not interfering with measurement of the detection layers.

The assay rod with the desired number of detection layers and the various desired known antigens impregnated within the detection layers can be used in any of the conventional ELISA-type test procedures. The following description will be limited to an ELISA-type detection system based upon an enzyme-linked antibody marker. Other ELISA-type detection systems such as the use of a fluorescent antibody marker may be used.

The support tube 26 and enclosed assay rod is attached to a suitable manifold and vacuum is applied to draw up the sample solution above the top of the assay rod 10. The support tube is then incubated for a sufficient time and at a sufficient temperature to allow antibodies within the solution to immunochemically react with their corresponding antigens present in the various detection layers. During incubation, a pulse vacuum source is preferably used to draw the solution up and down in the support tube 26 to assure complete mixing and agitation. After incubation, air pressure is applied to the top 26 of support tube 22 to blow out the test solution. The assay rod 10 is then rinsed a number of times by drawing out and expelling a suitable rinsing solution such as standard saline.

After rinsing, a suitable enzyme-linked second antibody, such as an antibody linked to horse-radish peroxidase (HRP) is drawn up into the tube 26. As is conventionally known, the HRP-linked antibody is chosen such that the HRP-linked antibody will attach to any antibodies which are bound to the detection layers by way of the known impregnated antigens. Typically, if the antibodies being tested for are human antibodies, the enzyme-linked detector antibody or second antibody is preferably an antibody directed against all human IgG antibodies, all human IgE antibodies, all human IgM antibodies or the detector antibody may be directed against all three types.

The detector or marker antibody is incubated at conventionally known temperatures and for conventionally known times followed by washing of the rod to remove any unbound detector antibodies. A color reagent for the HRP-linked antibody, such as diaminobenzidine plus hydrogen peroxide, is then added to the assay rod 10. The colored product produced by any HRP-linked antibody bound to the detection layers is trapped within the respective filter paper layers. The assay rod is then screened using a suitable optical detector to determine which detection layers have colored product. The system herein described can be adapted to the "sandwich assay" for the presence of multiple antigens. Antibodies specific for various antigens are attached to different detector layers and the rod constructed as before. A solution containing many possible antigens to be assayed is then incubated with the rod. Following washing, a solution is added which contains a mixture of marker-labelled antibodies directed against the same antigens as the antibodies attached to the detection layers. The assay is completed and read as before.

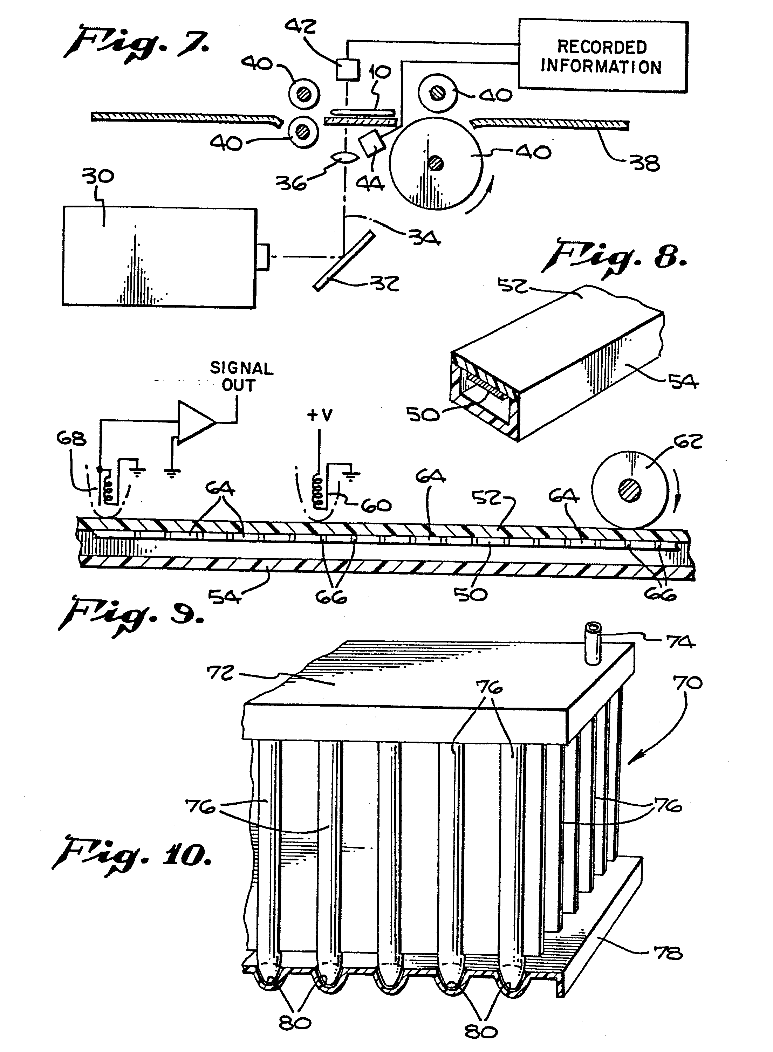

Fig. 7 is a schematic representation of a preferred screening system for "reading" the results of the immunoassay carried out using the assay rod. Basically, qualitative and quantitative information can be gained by measuring the amount of colored product found in each filter paper layer. This is done by measuring the attenuation of light passing through an individual filter paper layer as it moves between a light beam and a light detector. As shown in Fig. 7, a light beam is provided by laser 30 by way of mirror 32. The light beam 34 is passed through a suitable lens 36 to provide a beam having a width of 0.002 inches. The tube 10 is positioned on a guide plate 38 and moved past the light beam using a suitable drive wheel system 40. An axially sensor 42 produces an analog signal proportional to the amount of the light beam transmitted through rod 10. A back scatter sensor 44 is provided to register imperfections in the assay rod (principally air bubbles) which might produce artifacts in light transmission. The sensor 44 is also used to test the integrity and uniformity of each rod or channel before it is used in an immunoassay. The signals from sensors 42 and 44 are passed to a computer control unit. The computer control unit also controls the drive wheel assembly so that the signals received from sensors 42 and 44 can be correlated to each individual detector layer to thereby provide accurate measurement and identification of each layer. The system can be designed to provide both qualitative and quantitative information. Other conventional reading systems are of course possible so long as they provide discrete measurement of the transmitted, reflected or fluorescent light of each detector layer. Other optical detection methods, such as phosphorescence, light scattering and polarized light modulation, may also be used.

Another aspect of the present invention, is the use of a magnetizable marker which can be linked to a suitable antibody to provide a particularly desirable marker system for use in "reading" the numerous detector layers present in the preferred assay rods. Instead of tagging a suitable second antibody with an enzyme or fluorescent compound, the second antibody may be tagged with a magnetizable substance such as ferritin. The tagging of antibodies with ferritin and other magnetizable compounds such as colloidal iron, colloidal cobalt or proteinaceous microspheres containing iron oxide particles is well known with numerous magnetizable tags and methods for linking them to suitable antibodies being well known. (See - Ferritin-protein conjugates: Kishida, Y., Olsen, B.R., Berg, R.A. and Prockop, D.J. 1975, J. Cell Biol. 64, 331-339; Colloidal iron: Poynton, C.H., Dicket, K.A., Culbert, S., Frankel, L.S., Jagannath, S., and Reading, C.L. 1983, March 5. The Lancet pg. 524; and Microspheres containing iron oxide: Widder, K.J., Senyei, A.E., and Scarpelli, D.G. 1978. Proc. Soc. Exp. Biol. and Med. 58:141-146.)

Alternatively, magnetic ions can be deposited from a solution by the action of an enzyme conjugated to the second antibody. For example, many companies supply antibodies conjugated with Alkaline phosphatase. The presence of a enzyme can be detected and quantified with a standard histochemical staining procedure, the calcium-cobalt method (see Gomori, G. 1946. Amer. J. Clin. Path. 16, tec. sect. 7,177; or see any manual on enzyme histochemistry, e.g. Pearse, A.G.E., 1968. Histochemistry, Theoretical and Applied, 3rd edition, vol. 1 pp. 495-511, and pp. 710-711). In this reaction, the sites of enzyme activity are assayed by the presence of an organic phosphate ester in the presence of calcium ions, resulting in the deposition of insoluble calcium phosphate. Treatment with soluble cobalt nitrate produces a precipitate of cobalt phosphate, which has a high magnetic susceptibility. The assay rod fabricated for use when magnetizable markers are being used for detection, are basically the same as the previously described assay rods except that the spacing layers are preferably impregnated with iron oxide. Also, it is not necessary that the support tube or tape backing for the rod be of optical quality.

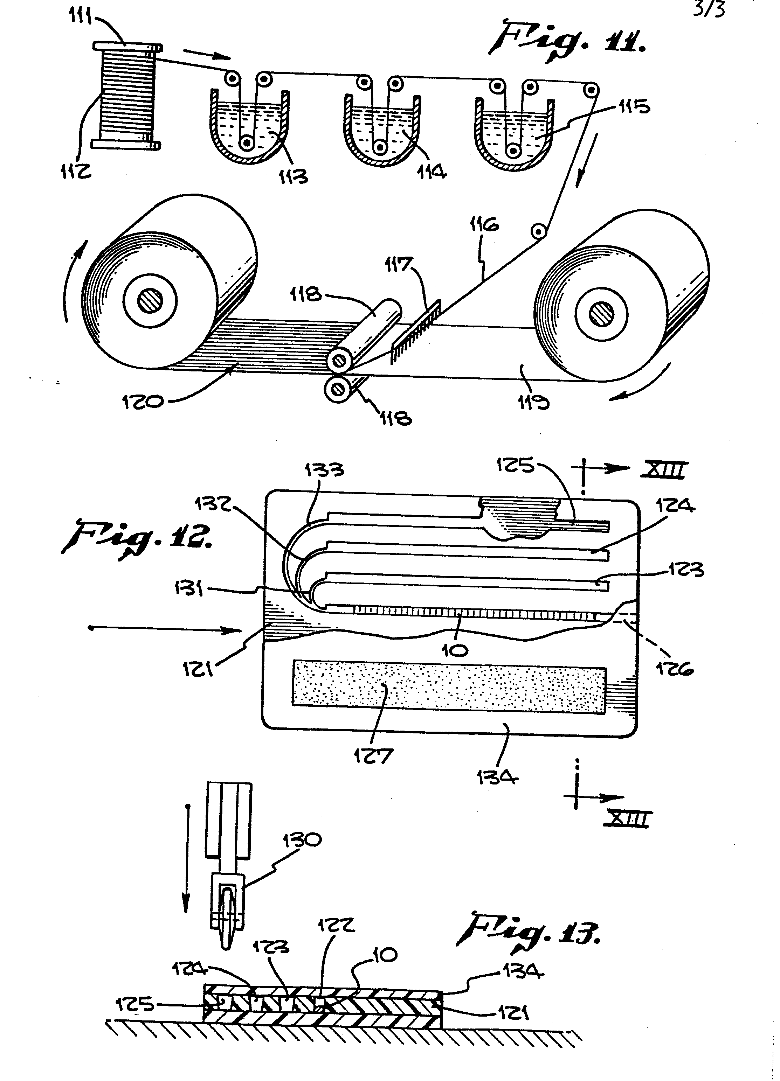

An assay rod for use in a magnetic detection system is shown generally at 50 in Fig. 8. The rod 50 is attached to a cover plate 52 (preferably plastic) which is sealed over a channel 54. Although only a single rod and channel arrangement is shown in Fig. 8, it is preferred that a channel plate (not shown) be used in which a series of parallel channels are formed in a single plate so that numerous rods 50 may be conveniently positioned for multiple sample testing and multiple rod scanning or measurement.

The procedure for screening solutions for particular antibodies is the same for assay rod 50 as for the previously described assay system. The only difference is that instead of using an enzyme-linked antibody to detect the presence of antibody indirectly bound to the detection layers, an antibody linked to a magnetizable marker is used. Instead of a colored product being bound to the detection layers, a magnetizable marker is present.

Fig. 9 is a schematic representation of a magnetic scanner which is used to detect the presence of any magnetizable antibody marker present in the detection layers. The magnetic scanning device is similar to conventional credit card readers. The magnetic scanner includes a stepping motor 62 which controllably passes the channel 54 and enclosed rod 50 past a write head 60. The write head 60 magnetizes any magnetizable marker present in the detection layers 64 of rod 50. The iron oxide present in spacer layers 66 is also magnetized. The rod 50 is then passed by a read head 68. The magnetized iron oxide and any magnetized marker will produce an analog signal from the read head 68 which can be fed directly into a computer for processing. The use of a verticle magnetic medium playback device, in which the 2 poles of the read and write heads are on opposite sides of the M.I. rod, will permit an increased packing density of probes and greater sensitivity than the more conventional system of Fig. 9. The "vertical recording" technique is available from Eastman Kodak or the 3M Company. The use of a magnetizable marker is especially well suited for reading assay rods having a large number of detection layers since it provides a particularly convenient means of direct input into a computer.

As is apparent from the above description, the immunoassay rod in accordance with the present invention provides a particularly convenient means for screening a test solution for a large number of antibodies using conventional techniques which were previously limited to simultaneous detection of only a few antibodies.

Instead of using filter paper detection layers and interleaved plastic layers, an alternative assay rod in accordance with the present invention can be fabricated from various threads such as nitrocellulose thread or other suitable thread such as nylon, cotton or the like. In general, the assay rod is fabricated by first treating the desired number of threads with the desired number of known antigens to provide antigen impregnated threads. The antigen impregnated threads are then aligned in parallel and stretched tight using a loom. A piece of adhesive tape or other backing material is then attached to the parallel threads. The tape may then be sectioned in a manner similar to the procedure previously shown in Fig. 6. Spacer threads of different color which are not impregnated with antigens may be placed in between the antigen impregnated threads if desired to provide easier identification and positive spacing of the antigen impregnated threads. In addition, the detection layers for the assay rods in accordance with the present invention may be produced by printing the known antigens in thin lines onto filter paper. Filter paper may then be cut into thin rods with the rods being applied to a suitable adhesive backing in a manner similar to the procedure used for antigen impregnated threads.

A method of fabrication of nylon monofilament threads coated with antigens or antibodies, which can be incorporated into an exemplary microassay rod is shown in Fig. 11. Depending upon the number of probes desired, one or more bobbins 111 containing nylon monofilament line 112 are set up. For simplicity, only one bobbin and monofilament nylon line are shown in Fig. 11. The thread 112 is first treated chemically by slowly pulling the thread 112 through a sensitizing solution 113 containing a bifunctional reactive substance which covalently attaches to the nylon surface. The filament 112 is then slowly pulled through the antigen or antibody solution 114 which reacts covalently with the other half of each bifunctional reactive substance. The filament 112 is then finally passed through a wash bath 115 where a wash solution is provided to react with any remaining reactive substances on the nylon.

The chemistry of attaching proteins to nylon threads has been worked out (Sundaram, P.V. & Hornby, W.E. 1970. FEBS Lett. 10:325-327, and Sundaram, P.V. 1977, in Biomedical Applications of immobilized enzymes and proteins. Thang, T.M.S. ed. pp. 317-340. Plenum Press, New York.). Gordon, N.C., Shihita, M., & Hall, P.F. 1974. J. Ultrastruct. Res. 47:285-295.

Alternatively, the immunoreagent can, using a similar process, be attached by non-covalent adsorption to a thread made from a suitable polymer. Catt, K. & Tregear, G.W. 1967. Science 158:1570-1572.)

The treated thread 116 is then pulled through a weaving comb 117 which aligns each thread in parallel. The thread 116 is then pressed by suitable rollers 118 or other device onto adhesive tape 119. The tape 119 with thread 116, plus other desired threads shown generally at 120 may then be sectioned in a manner similar to the procedure previously shown in Fig. 6.

When numerous samples are to be tested simultaneously, the immunoassay rods and support tubes may be arranged in a manifold as shown in Fig. 10. The manifold shown generally at 70 includes an upper manifold plate 72 which has a central vacuum attachment 74 which can be connected to a vacuum system to draw samples up into the support tubes 76. The manifold can be positioned over a standard microtiter tray as shown at 78 for the simultaneous analysis of numerous different samples present in the individual wells 80 in microtiter tray 78. Washing and subsequent treatment steps are carried out by placing the manifold 70 over additional microtiter plates having the desired wash solution or marker antibody etc. in the microtiter plate wells.

An example of an assay system based upon the microassay rod 10 in accordance with the present invention is shown in Figs. 12 and 13. Referring to Fig. 12, a plastic card 121 is provided which has a series of closed channels 122, 123, 124 and 125. The microassay rod 10 is positioned in channel 122. The sample to be tested is inserted into channel 122 through opening 126 or by other suitable injection. The card 121 includes a magnetic or optically coded information strip of the type conventionally used on credit cards which contains all of the information pertaining to the desired assay and analysis of results. After loading of the sample into channel 122, the card is fed into a card reading device, which reads the magnetically or optically coded information on strip 127 which identifies, among other things, the probes on the rod, the method of their assay, the previously calibrated data for each probe and information on the analysis of the quantitative results. The card is then incubated according to information read from strip 127 for the desired incubation time. The card is then passed under a solenoid operated roller 130 which squeezes the test sample out of channel 122 through opening 126. The card is then moved automatically so that channel 123 is placed under solenoid actuated roller 130. The roller 130 is then used to force wash solution from channel 123 through conduit 131 into channel 122. After a suitable time period, the wash solution may then be removed from channel 122 by way of the solenoid operated roller 130. Then, the card is then positioned so that channel 124 is placed under roller 130. The card and/or roller 130 are then moved so that the desired chromagenic second antibody or other detecting solution is forced from channel 124 through column 132 into channel 122. After a sufficient time to allow the chromogenic second antibody to react with the rod 10, the chromogenic solution is removed from channel 122 by roller 130. Wash solution in channel 125 is then forced into channel 122 by roller 130 which forces this solution through conduit 133 and into contact with rod 10. Although the card is shown utilizing only four channels, it is possible to utilize as many channels as desirable or necessary to provide the desired analysis. The cover layer 134 for card 121 (see Fig. 13) is preferably made from a suitably flexible material which can be deformed by roller 130 so that the various solutions in channels 122-125 can be moved by the peristaltic action induced by roller 130. Also, more than one solenoid operated roller may be used if desired.

Examples of practice are as follows:

An assay rod having nine detection layers was prepared as described above from filter paper and Surlyn plastic. The rod was approximately 100 microns thick, 0.5mm wide and 4mm long. The rod was mounted within a round glass pipette, 13 cm long, 50 microliter volume. The rod was used to detect the presence of a purified mouse monoclonal antibody directed against human Chorionic Gonadotrophin. A 100 microliter test solution containing 10 nanograms/ml was tested. The nine detection layers or probes were either untreated (blanks) or contained approximately 100 ng of various mixed or purified proteins. The order of detection layers in the rod was: Blank, human transferrin, bovine serum albumin, human Chorionic Gonadotrophin antigen, fetal calf serum, luteinizing hormone, blank, bank, blank. Following incubation with the test solution containing the Chorionic Gonadotrophin antibody, its binding was detected essentially by following the procedures of a Vectastain kit (available from Vector Laboratories, Inc.), containing a biotinilated second antibody directed against mouse IgG heavy and light chains. A horse radish peroxidase color development reagent (BioRad Laboratories) was used. The only detection layer which showed a positive stain was the layer containing the human Chorionic Gonadotrophin.

A second rod having 79 detection layers and a length of 18mm was prepared as set forth in the first example. The detection layers contained protein extracts from various parts of the brain and other tissues of rat, mouse, chicken and human origin. The above rod was used to detect and characterize monoclonal antibodies present in the supernatants of hybridoma cultures. This assay detected various monoclonal antibodies with differing specificities determined by the MI rod assay correlated with results obtained by binding the monoclonal antibodies directly to tissue slices, using conventional immunohistological techniques.

As is apparent from the preceding description, the assay rod of the present invention is a valuable new analytical tool for conducting a large number of tests simultaneously on a small sample. In an addition to the above described features and advantages, a single assay rod can use some of its detector layers as internal standards, internal positive controls, and internal negative controls. This greatly aids in quantitation, and in detecting false negatives and false positives. For example, these controls would be as follows:

a. Internal standard: if the second antibody had an enzyme marker, then a known amount of that enzyme could also be incorporated into one of the detection layers. The color development in this layer would be a standard used to quantitate the amount of enzyme present in the other layers.

b. Internal positive controls. For example, the unlabeled second antibody could be incorporated into a detection layer. Then, if any first antibody was in the test solution, and the assay was working properly, this layer should stain.

c. Internal negative controls. Layers containing substances to which antibodies should not bind. If these layers stained during the assay, then something is amiss.

Further advantages include the fact that different concentrations of the same antigen can be applied to different layers, providing an internal dose-response curve. In addition, the microassay rod serves as a small and conveniently stored permanent record of the test results.

Having thus described exemplary embodiments of the present invention, it should be noted by those skilled in the art that the within disclosures are exemplary only and that various other alternatives, adaptations and modifications may be made within the scope of the present invention. For example, the microassay system is easily adapted to the detection of nucleic acid hybridization (in fact nitrocellulos filters first gained general use for this purpose). There are now a multitude of cloned genes to be used as probes so that the microassay system has great utility in human genetic screening and in the identification and typing of microorganisms by the hybridization of their genetic material, to mention only two applications. In addition, there are a number of other molecular interactions between two substances which can be exploited to test for the presence of one or the other substances such as:

| Molecule | To test for |
| --- | --- |
| 1. Receptor | Hormone, transmitter, synethetic ligand, drug |
| 2. Lectin | Sugars or polysaccharides |
| 3. Enzyme | Substrates or cofactors |

Accordingly, the present invention is not limited to the specific embodiments as illustrated herein and is only limited by the following claims.

CLAIMS

1. An microassay rod adapted for use in the detection of the presence of substances in test solution which engage in binding reactions comprising:

a column having a plurality of spaced detection layers, at least one of said detection layers including a binding amount of a known binding reagent bound thereto, said binding reagent being capable of specifically binding with one of said substances being tested for to bind said substance to said detection layer to thereby provide for detection of the presence of said substance in said test solution; and

means for positioning said detection layers at said spaced locations in said column to form said microassay rod.

2. A microassay rod according to claim 1 wherein said binding reagent bound to said detection layer is a known antigen and said substance being tested for is an antibody.

3. A microassay rod according to claim 1 wherein said immunoreactive reagent bound to said detection layer is a known antibody and said substance being tested for is an antigen.

4. A microassay rod according to claim 1 in which said detection layers have bottom and top surfaces and wherein said means for positioning said detection layers includes spacer layers having top and bottom surfaces, said spacer layers being located between said spaced detection layers and means for adhering said detection layer top surface to said spacer layer bottom surface and said detection layer bottom surface to said spacer layer top surface to thereby provide a laminated microassay rod having alternating detection and spacer layers.

5. A microassay rod according to claim 1 wherein said detection layer is made from cellulose filter paper and said spacer layer is a plastic.

6. A microassay rod according to claim 1 wherein said detection layer comprises a thread which is capable of binding said binding reagent thereto.

7. A method for detecting the presence of substances in a test solution comprising the steps of:

providing an microassay rod having a plurality of spaced detection layers located at fixed spaced locations to provide a column of discrete detection layers, wherein at least one of said detection layers includes a binding amount of a known binding reagent bound thereto, said binding reagent being capable of specifically binding with one of said substances being tested for to bind said substance to said detection layer;

applying said test solution to said microassay rod in a sufficient amount and for a sufficient time to allow binding reaction of said bound binding reagent with any of said substance in said test solution; and

detecting the presence of any of said substance bound to said binding reagent.

8. A method according to claim 7 wherein said step of detecting the presence of said substance comprises:

treating said rod with a marker which binds selectively to said substance and which has an identifiable characteristic for a sufficient time and at a sufficient temperature to bind said marker to said bound binding substance; and

measuring said identifiable characteristic substance to thereby detect the presence of said substance.

9. A method according to claim 8 wherein said identifiable characteristic is an enzyme linked to said marker.

10. A method according to claim 8 wherein said identifiable characteristic is flourescence.

11. A method according to claim 8 wherein said identifiable characteristic is a magnetizable moiety linked to said marker.

12. A microassay rod according to claim 4 wherein said spacer layers are magnetizable.

13. An immunoassay rod adapted for use in the simultaneous detection of the presence of a plurality of immunoreactive substances comprising:

a laminated column of alternating detection layers and spacer layers, said detection layers including immunoreactive reagents bound thereto for detecting the presence of said immunoreactive substances.

14. An immunoassay rod according to claim 13 wherein said detection layer is made from cellulose filter paper, a cellulose nitrate filter paper, or nylon filter paper.

15. An immunoassay rod according to claim 13 wherein said detection layer is made from a filamentous or monofilament thread.

16. An immunoassay rod according to claim 13 wherein said spacer layer is made from a plastic material.

17. A microassay apparatus comprising an microassay rod according to claim 1 housed within a support tube, said support tube having two open ends to facilitate transfer of solutions into contact with said rod.

18. A microassay assay manifold apparatus comprising a plurality of microassay rods and support tubes according to claim 17 connected in parallel relationship to provide simultaneous testing of a plurality of test solutions.

19. A method for detecting the presence of a substance in a test solution which engages in binding reactions comprising the step of treating said substance with a known binding reagent, said binding reagent containing a magnetizable marker and capable of specifically binding with one of said substances being tested for.

20. A method according to claim 19 wherein said immunoreactive reagent is an antibody or antigen labeled with a magnetizable marker.

21. A method according to claim 20 wherein said magnetizable marker is selected from the group consisting of ferritin, colloidal cobalt, colloidal iron, and microspheres impregnated with iron oxide particles.

22. A microassay system comprising an microassay rod according to claim 1 and means for measuring the presence of binding reaction in each of said detection layers.

23. A microassay system according to claim 22 wherein said measuring means includes means for optically analyzing each of said detection layers.

24. A microassay system according to claim 22 wherein said measuring means includes means for magnetically analyzing each of said detection layers.

25. A microassay system comprising:

a card having a longitudinal channel therein defining a reaction zone for housing a microassay rod according to claim 1, said channel having an inlet and an outlet, said card further having one or more other longitudinal channels for housing reagent and wash solutions, said reagent and wash channels having an outlet end;

conduit means for connecting the outlets of the reagent and wash channels to the inlet of the rod housing channel; and

means for selectively transferring said reagent and wash solutions from the reagent and wash channels to the rod channel to provide reagents and wash solutions for reaction with and washing of the microassay rod in the rod channel.

Fig. 1.
10
12
12
14
12
14
12
14
Fig. 2.
26
22
10
III
III
24
28
Fig. 3.
10
22
Fig. 4.
16
14
12
18
20
14
12
16
Fig. 5.
18
20
Fig. 6.

Fig. 7.
42
40
10
RECORDED
INFORMATION
40
40
40
38
36
44
30
34
32
Fig. 8.
52
54
50
SIGNAL
OUT
+V
68
64
60
64
52
64
62
54
66
50
66
Fig. 9.
74
72
76
70
76
76
Fig. 10.
78
80
80

Fig. 11.
111
112
113
114
115
116
117
118
118
119
120
Fig. 12.
125
XIII
133
132
124
123
131
10
121
126
127
134
XIII
130
Fig. 13.
122
123
10
124
134
125
121

European Patent Office

# EUROPEAN SEARCH REPORT

0139373

Application number: EP 84305455.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US - A - 4 219 335 (EBERSOLE) * Abstract * | 1,11, 12,20, 21 | G 01 N 33/543<br>G 01 N 33/535<br>G 01 N 33/532<br>G 01 N 33/483 |
| Y | US - A - 4 363 874 (GREENQUIST) * Abstract * | 1,10 | |
| D,A | ANALYTICAL BIOCHEMISTRY, vol. 119, no. 1, January 1, 1982<br>R. HAWKES, E. NIDAY and J. GORDON "A Dot-Immunobinding Assay for Monoclonal and other Antibodies" pages 142-147<br>* Abstract * | 1,7,8 | |

TECHNICAL FIELDS SEARCHED (Int. Cl.4): G 01 N 33/00

The present search report has been drawn up for all claims

| Place of search | Date of completion of the search | Examiner |
|---|---|---|
| VIENNA | 03-11-1984 | SCHNASS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82